# EUROPÄISCHE PATENTSCHRIFT

(11) **EP 1 584 332 B1**
(45) Veröffentlichungstag und Bekanntmachung des Hinweises auf die Patenterteilung: **02.12.2009**
(21) Anmeldenummer: 05104551.6
(22) Anmeldetag: 29.09.2000
(51) Int. Cl.: A61K 8/97, A61Q 19/10

(54) **Verfahren zum Bleichen von Naturstoffen**
Process for bleaching natural substances
Procédé pour blanchir des substances naturelles

(30) Priorität: 08.12.1999 DE 19959238
(43) Veröffentlichungstag der Anmeldung: 12.10.2005
(62) Teilanmeldung aus: 00121183.8
(73) Patentinhaber: Peter Greven Fett-Chemie GmbH & Co.KG, 53902 Bad Münstereifel (DE)
(72) Erfinder: Stolz, Hermann-Josef, Dr., 53902 Bad Münstereifel (DE); Börnicke, Robert, 53902 Bad Münstereifel (DE)
(74) Vertreter: Schreiber, Christoph

(56) Entgegenhaltungen:
- DE-A1- 1 519 186
- DE-A1- 4 038 076

## Beschreibung

Die Erfindung betrifft ein Verfahren zum Bleichen von Naturstoffen die als Abrasivum für Reinigungsmittel eingesetzt werden können. Ein ideales Abrasivum soll abgerundete Körner haben, eine spezifische Dichte von weniger als 1 g/cm³ und biologisch abbaubar sein.

Die zuerst verwendeten Abrasiva waren auf Basis von Quarzsand und Bimsmehl. Danach sind Abrasiva mit Holzmehl entwickelt worden, die dann durch Kunststoffgranulate ersetzt wurden. In der letzten Zeit verwendet man zunehmend Bioreibkörper.

Quarzsand und Bimsmehl haben den Nachteil, daß die Körner hart und scharfkantig sind und eine Dichte von mehr als 1 g/cm³ aufweisen. Die Scharfkantigkeit der Körner hat zur Folge, daß Quarzsand und Bimsmehl eine relativ schlechte Hautverträglichkeit haben. Die relativ hohe Dichte hat den Nachteil, daß die Quarzsand- und Bimsmehle zur Verstopfung von Abflüssen durch Sedimentation führen können.

Deshalb verwendete man zunehmend Holzmehle. Die Holzmehle sind bezüglich der Hautverträglichkeit wesentlich besser als Quarzsand und Bimsmehl, und da sie eine spezifische Dichte von weniger als 1 g/cm³ haben, ist eine Verstopfung von Abflüssen durch Sedimentation nicht mehr zu befürchten. Nachteilig bei der Verwendung von Holzmehlen ist der mögliche Gehalt an Kolophonium und bestimmten Terpenen, die zu Allergien führen können. Ein weiterer Nachteil ist, daß Holzstaub im Verdacht steht, krebserregend zu sein, so daß strenge Sicherheitsvorkehrungen bei der Herstellung von Holzmehlen zu beachten sind.

Heute werden die meisten Abrasiva auf der Basis von Polyurethan- oder Polyethylenmehl hergestellt. Beide Mehle sind sehr vorteilhaft, da sie eine wenig scharfkantige Struktur haben, eine Dichte, die weniger als 1 g/cm³ beträgt, eine einstellbare Kornverteilung, eine einstellbare physikalische Härte und eine konstante Produktqualität durch reproduzierbare Herstellungsprozesse aufweisen. Allerdings sind Kunststoffgranulate sehr schwer biologisch abbaubar und können nur thermisch verwertet werden. Kunststoffmehle sind daher in fast allen Belangen ideale Abrasiva, haben aber Nachteile in bezug auf Entsorgung, Umweltschutz und auch Abbaubarkeit.

Bei der Suche nach Alternativen für biologisch abbaubare Stoffe, und insbesondere Naturstoffe, werden Bioreibköper auf Basis von Naturschalenmehl verwendet. Walnußschalenmehl weist eine relativ hohe Dichte auf, die nahe bei 1 g/cm³ liegt. Eine Sedimentation in Abflüssen kann nur durch optimierte Kornverteilung vermieden werden. Walnußschalenmehl weist außerdem ein hohes Allergiepotential auf. Die Verwendung von Nußschalen- und Kernmehlen als Abrasivum für kosmetische Zwecke ist in EP 0 559 696 beschrieben.

Aus dem geschilderten Stand der Technik wird deutlich, daß noch immer ein dringender Bedarf an einem Abrasivum besteht, das vollkommen abbaubar ist, ein niedriges Allergiepotential aufweist, eine niedrige Dichte besitzt und daher als Alternative zum Naturschalenmehl verwendet werden kann.

Maismehl wird auch heute schon in unbehandelter Form als Abrasivum eingesetzt, besitzt jedoch erhebliche Nachteile hinsichtlich Optik, Geruch, Hygiene und Verkeimungsgefahr. Daher war die Verwendung von Maiskolbenmehl in Hautreinigungspräparaten bisher sehr begrenzt. Der vorliegenden Erfindung liegt die Aufgabe zugrunde, einen abrasiven Stoff für Hautreinigungsmittel auf Basis von Maiskolbenmehl bereitzustellen, der die in dem Stand der Technik geschilderten Nachteile nicht aufweist und neue Möglichkeiten bei der Verwendung von Maiskolbenmehl eröffnet. Insbesondere soll der abrasive Stoff gute Eigenschaften besitzen, ohne Zusatz oder gegebenenfalls nur mit einem minimalen Zusatz von weiteren chemischen Mitteln.

Diese Aufgabe wird durch die Verfahren gemäß den Ansprüchen 1, 8 und 9 gelöst.

Es hat sich überraschenderweise herausgestellt, daß durch das Bleichen von Maiskolbenmehl ein hochwertiger abrasiver Stoff hergestellt werden kann. Die vorliegende Erfindung betrifft somit ein Verfahren zum Bleichen von Naturstoffen, die als abrasive Stoffe für kosmetische Präparate, die gegebenenfalls Tenside, Seifen oder andere Emulgatoren, organische Lösungsmittel oder Öle sowie gegebenenfalls Verdikkungsmittel, Gerüststoffe, Rückfettungsmittel, Parfümstoffe, Konservierungsmittel, Farbstoffe und Antioxidantien enthalten, geeignet sind.

Das Maiskolbenmehl wurde bisher üblicherweise als Füllstoff verwendet. Eine Verwendung von Maiskolbenmehl ist in OS DE 39 14 969 A1, OS DE 36 24 468 A1, DE 32 07 036 C2, DE 32 44 452 C2 beschrieben. Maiskolbenmehl besitzt zwar eine zum Teil faserige Struktur, besteht im Gegensatz zum Walnußmehl aber nur zu etwa 5 bis 10% aus Lignin und ca. 90% aus Zellulose. Die Reib- oder Reinigungswirkung von Maiskolbenmehl ist geringer als beim Walnußschalenmehl. Die Reinigungswirkung ist aber immer noch ausreichend, da die Verschmutzungsgrade in der Industrie nicht mehr so hoch sind und ein gutes Hautreinigungsmittel eher eine bessere Hautverträglichkeit aufweisen muß. Das Maiskolbenmehl ist hautverträglicher als Walnußschalenmehl. Die spezifische Dichte von Maiskolbenmehl ist geringer als die spezifische Dichte von Walnußschalenmehl. Damit weist Maiskolbenmehl große Vorteile gegenüber dem Stand der Technik auf.

Das Bleichen von Maiskolbenmehl ist in dem Stand der Technik nicht beschrieben. Es konnte daher überraschend festgestellt werden, daß durch das Bleichen von Maiskolbenmehl dessen Eigenschaften in vorteilhafter Weise drastisch verändert werden. Das hängt damit zusammen, daß das Maiskolbenmehl während des Bleichvorgangs quillt. Durch das Bleichen wird nicht nur das Aussehen verbessert, sondern auch die Struktur und die Korneigenschaften werden verändert. Des weiteren werden auch die gegebenenfalls vorhandenen Keime abgetötet. Damit ist das gebleichte Maiskolbenmehl dem ungebleichten Maiskolbenmehl in mehrfacher Hinsicht überlegen.

Eine Aufgabe der Erfindung ist es, ein einfaches Verfahren zur Herstellung von Abrasiva vorzuschlagen. Diese Aufgabe wurde durch das einstufige Herstellungsverfahren gemäß Anspruch 9 gelöst.

Schließlich ist es die Aufgabe der Erfindung, ein Verfahren zur Herstellung von kosmetischen Zubereitungen, insbesondere Handwasch- und Handreinigungsmittel, gemäß Anspruch 9 bereitzustellen.

Nachstehend wird die Herstellung von dem erfindungsgemäßen Abrasivum beschrieben.

Zur Herstellung des erfindungsgemäßen Abrasivums können beispielsweise folgende Bestandteile verwendet werden :

| | |
|---|---|
| Wasser | 54,82% |
| Maiskolbenmehl | 10,00% |
| Xanthan | 0,20% |
| Bentonit | 1,00% |
| Wasserstoffperoxid 35% | 1,00% |
| Natronlauge 50% | 1,20% |
| Zitronensäure | 0,30% |
| Titandioxid | 0,50% |
| PEG 7-Glycerylcocoat | 2,00% |
| Cocamidopropylbetain | 9,00% |
| Natriumlaurylethersulfat 70% | 19,60% |
| Zitronenschalenduft | 0,20% |
| Bronopol 30% | 0,18% |

Wasser, Maiskolbenmehl, Xanthan und Bentonit werden gemischt (wobei der pH-Wert der Mischung ca. 7,4 beträgt). Nach der Quellung von Maiskolbenmehl wird Wasserstoffperoxid zugegeben und untergemischt. Das Bleichen wird mit Natronlauge aktiviert. Nach der Zugabe von Natronlauge liegt der pH-Wert bei ca. 11,9. Die Mischung wird so lange gerührt, bis der pH-Wert deutlich abgefallen ist und vorzugsweise weniger als 10 ist. Erst dann wird mit Zitronensäure das restliche Wasserstoffperoxid ausgetrieben. Nach der Bleichung werden der Bleichmischung die restlichen Rohstoffe zugefügt.

Es ist selbstverständlich, daß statt Natronlauge auch andere Hydroxide verwendet werden können. Statt der Zitronensäure können auch andere Säuren verwendet werden. Auch die Hilfsstoffe können beliebig variiert werden.

Das erfindungsgemäße Verfahren weist gegenüber den herkömmlichen Bleichverfahren den Vorteil auf, daß die Herstellung von dem Abrasivum in einem Schritt erfolgt. Daher muß das gebleichte Mehl nicht abfiltriert, anschließend getrocknet und dann nachbehandelt werden, was sich positiv auf Rohstoff- und Energieeinsatz auswirkt und Ressourcen schont. Alle Schritte werden erfindungsgemäß einstufig durchgeführt. Das erfindungsgemäße Verfahren kann auch für andere Naturstoffe verwendet werden, wobei die Verwendung für Maismehl große Vorteile hat, da Maismehl im Bleichprozeß quillt. Die eventuell im Rohstoff vorhandenen Keime werden durch den Prozeß vollständig abgetötet. Das wesentliche Merkmal des Verfahrens ist, daß eine Säure der Bleichmischung erst bei einem pH-Wert von 10 oder weniger zugegeben wird. Damit ist sichergestellt, daß eine vollständige Zerstörung des Peroxids am Ende des Prozesses erfolgt, was durch Titration nachgewiesen werden kann.

Durch das erfindungsgemäße Verfahren lassen sich helle, saubere und kosmetisch einwandfreie Hautreinigungsmittel erhalten. Der Zusatz von Aufhellungsmitteln ist nicht notwendig. Der Gehalt an Titandioxid ist sehr gering und daher fast zu vernachlässigen. Die gebleichten Mehle sind biologisch vollständig abbaubar und weisen ein niedriges Allergiepotential auf. Die Gefahr, daß die Verwendung von Maiskolbenmehl zur Verstopfung von Abflüssen führen kann, besteht nicht.

## Patentansprüche

1. Verfahren zum Bleichen von Naturstoffen, **dadurch gekennzeichnet, dass** Wasser, Naturstoffmehl und gegebenenfalls andere Hilfsstoffe gemischt werden, dass Wasserstoffperoxid und Alkalihydroxid der Mischung zugesetzt werden, und - Wenn der pH-Wert der Mischung auf einen Wert von 10 oder weniger absinkt - eine Säure zugesetzt wird bis Wasserstoffperoxid nicht mehr nachzuweisen ist, und dann andere Hilfsstoffe der Mischung zugesetzt werden, wobei das gemäß dem Verfahren erhältliche gebleichte Mehl nicht abfiltriert, anschließend getrocknet und dann nachbehandelt wird.

2. Verfahren nach Anspruch 1, **dadurch gekennzeichnet, dass** das Alkalihydroxid Natronlauge ist.

3. Verfahren nach einem der Ansprüche 1 oder 2, **dadurch gekennzeichnet, dass** die Säure Zitronensäure ist.

4. Verfahren nach einem der Ansprüche 1 bis 3, **dadurch gekennzeichnet, dass** die einer nach Anspruch 1 erhaltenen Mischung zugesetzten anderen Hilfsstoffe Rohstoffe zur Herstellung eines Abrasivums für kosmetische Präparate sind.

5. Verfahren nach Anspruch 4, **dadurch gekennzeichnet, dass** die Rohstoffe ausgewählt werden aus der Gruppe bestehend aus Tensiden, Seifen oder anderen Emulgatoren, organischen Lösungsmitteln, Ölen, Verdickirngsmittein, Gerüststoffen, Rückfettungsmitteln, Parfümstoffe, Konservierungsmittel, Farbstoffe und Antioxidantien.

6. Verfahren nach Anspruch 4 oder 5, **dadurch gekennzeichnet, dass** das kosmetische Präparat ein Hautreinigungsmittel ist.

7. Verfahren nach einem der Ansprüche 1 bis 6, **dadurch gekennzeichnet, dass** das Naturstoffmehl ein Malskolbenmehl ist.

8. Verfahren zum Bleichen von Naturstoffen und zur Abtötung vorhandener Kelme, **dadurch gekennzeichnet, dass** Wasser, Naturstoffmehl und gegebenenfalls andere Hilfsstoffe gemischt werden, dass Wasserstoffperoxid und Alkalihydroxid der Mischung zugesetzt werden, und - wenn der pH-Wert der Mischung auf einen Wert von 10 oder weniger absinkt - eine Säure zugesetzt wird bis Wasserstoffperoxid nicht mehr nachzuweisen ist, und dann andere Hilfsstoffe der Mischung zugesetzt werden, wobei das gemäß dem Verfahren erhältliche gebleichte Mehl nicht abfiltriert, anschließend getrocknet und dann nachbehandelt wird.

9. Verfahren zum Herstellen eines kosmetischen Präparates **dadurch gekennzeichnet, dass** Wasser, Naturstoffmehl und gegebenenfalls andere Hilfsstoffe gemischt werden, dass Wasserstoffperoxid und Alkalihydroxid der Mischung zugesetzt werden, und - wenn der pH-Wert der Mischung auf einen Wert von 10 oder weniger absinkt - eine Säure zugesetzt wird bis Wasserstoffperoxid nicht mehr nachzuweisen ist, und dann andere Hilfsstoffe, ausgewählt aus der Gruppe bestehend aus Tensiden, Seifen oder anderen Emulgatoren, organischen Lösungsmitteln, Ölen, Verdickungsmitteln, Gerüststoffen, Rückfettungsmitteln, Parfümstoffe, Konservierungsmittel, Farbstoffe und Antioxidantien der Mischung zugesetzt werden, wobei das gemäß dem Verfahren erhältliche gebleichte Mehl nicht abfiltriert, anschließend getrocknet und dann nachbehandelt wird.

## Claims

1. A process for bleaching natural substances, **characterized in that** water, a meal of a natural substance and optionally other auxiliaries are mixed, hydrogen peroxide and alkali hydroxide are added to the mixture, and when the pH of the mixture has decreased to a value of 10 or less, an acid is added until hydrogen peroxide can no longer be detected, followed by adding other auxiliaries to the mixture, wherein steps of filtering off the bleached meal obtainable by the process, drying it subsequently and then aftertreating it are not performed.

2. The process according to claim 1, **characterized in that** said alkali hydroxide is aqueous sodium hydroxide.

3. The process according to either of claims 1 or 2, **characterized in that** said acid is citric acid.

4. The process according to any of claims 1 to 3, **characterized in that** said other auxiliaries added to a mixture obtained according to claim 1 are raw materials for preparing an abrasive for cosmetic preparations.

5. The process according to claim 4, **characterized in that** said raw materials are selected from the group consisting of surfactants, soaps or other emulsifiers, organic solvents, oils, thickening agents, builders, refatting agents, perfumes, preservatives, colorants and antioxidants.

6. The process according to claim 4 or 5, **characterized in that** said cosmetic preparation is a skin-cleansing agent.

7. The process according to any of claims 1 to 6, **characterized in that** said meal of a natural substance is a corn-cob meal.

8. A process for bleaching natural substances and killing existing germs, **characterized in that** water, a meal of a natural substance and optionally other auxiliaries are mixed, hydrogen peroxide and alkali hydroxide are added to the mixture, and when the pH of the mixture has decreased to a value of 10 or less, an acid is added until hydrogen peroxide can no longer be detected, followed by adding other auxiliaries to the mixture, wherein steps of filtering off the bleached meal obtainable by the process, drying it subsequently and then aftertreating it are not performed.

9. A process for preparing a cosmetic preparation, **characterized in that** water, a meal of a natural substance and optionally other auxiliaries are mixed, hydrogen peroxide and alkali hydroxide are added to the mixture, and when the pH of the mixture has decreased to a value of 10 or less, an acid is added until hydrogen peroxide can no longer be detected, followed by adding other auxiliaries selected from the group consisting of surfactants, soaps or other emulsifiers, organic solvents, oils, thickening agents, builders, refatting agents, perfumes, preservatives, colorants and antioxidants to the mixture, wherein steps of filtering off the bleached meal obtainable by the process, drying it subsequently and then aftertreating it are not performed.

## Revendications

1. Procédé de blanchiment de matières naturelles, **caractérisé en ce que** de l'eau, de la farine de substance naturelle et éventuellement d'autres matières auxiliaires sont mélangées, que du peroxyde d'hydrogène et de l'hydroxyde alcalin sont ajoutés au mélange, et - lorsque la valeur de pH du mélange diminue à une valeur de 10 ou moins - un acide est ajouté jusqu'à ce que le peroxyde d'hydrogène ne soit plus décelable, et ensuite d'autres matières auxiliaires sont ajoutées au mélange, dan lequel la farine blanchie disponible conformément au procédé est séparée par filtration, immédiatement séchée et est ensuite retraitée.

2. Procédé selon la revendication 1, **caractérisé en ce que** l'hydroxyde alcalin est de la lessive de soude.

3. Procédé selon les revendications 1 ou 2, **caractérisé en ce que** l'acide est de l'acide citrique.

4. Procédé selon les revendications 1 à 3, **caractérisé en ce que** selon la revendication 1 l'une des matières auxiliaires ajoutées obtenues par mélange sont des matières premières pour la fabrication d'un abrasif destiné à des préparations cosmétiques.

5. Procédé selon la revendication 4, **caractérisé en ce que** les matières premières sont choisies dans le groupe formé par les tensioactifs, les savons ou autres émulsifiants, solvants organiques, huiles, épaississants, adjuvants de lavage, agents nourrissants, substances aromatiques, agent conservateur, colorants et antioxydants.

6. Procédé selon les revendications 4 ou 5, **caractérisé en ce que** la préparation cosmétique est un produit de nettoyage de la peau.

7. Procédé selon les revendications 1 à 6, **caractérisé en ce que** la farine de substance naturelle est une farine d'épi de maïs.

8. Procédé de blanchiment de substances naturelles et d'étouffement de germes existants, **caractérisé en ce que** de l'eau, de la farine de substance naturelle et éventuellement d'autres matières auxiliaires sont mélangées, que du peroxyde d'hydrogène et de l'hydroxyde alcalin sont ajoutés au mélange, et - lorsque la valeur de pH du mélange diminue à une valeur de 10 ou moins - un acide est ajouté jusqu'à ce que le peroxyde d'hydrogène ne soit plus décelable, et ensuite d'autres matières auxiliaires sont ajoutées au mélange, dans lequel la farine blanchie disponible conformément au procédé est séparée par filtration, immédiatement séchée et est ensuite retraitée.

9. Procédé de fabrication d'une préparation cosmétique **caractérisé en ce que** l'eau, la farine de substance naturelle et éventuellement d'autres matières auxiliaires sont mélangées, que le peroxyde d'hydrogène et l'hydroxyde alcalin sont ajoutés au mélange, et - lorsque la valeur de pH du mélange diminue à une valeur de 10 ou moins - un acide est ajouté jusqu'à ce que le peroxyde d'hydrogène ne soit plus décelable, et ensuite d'autres matières auxiliaires, choisies dans le groupe formé par les tensioactifs, les savons ou autres émulsifiants, solvants organiques, huiles, épaississants, adjuvants de lavage, agents nourrissants, substances aromatiques, agents conservateurs, colorants et antioxydants sont ajoutés au mélange, dans lequel la farine blanchie disponible conformément au procédé est séparée par filtration, immédiatement séchée et est ensuite retraitée.
